# EUROPÄISCHE PATENTSCHRIFT

(11) **EP 1 555 008 B1**
(45) Veröffentlichungstag und Bekanntmachung des Hinweises auf die Patenterteilung: **16.03.2011**
(21) Anmeldenummer: 05100184.0
(22) Anmeldetag: 13.01.2005
(51) Int. Cl.: A61K 6/00

(54) **Mittel zum Bleichen von Zähnen**
Tooth bleaching composition
Composition de blanchiment des dents

(30) Priorität: 15.01.2004 DE 202004000552 U
(43) Veröffentlichungstag der Anmeldung: 20.07.2005
(73) Patentinhaber: Ivoclar Vivadent AG, 9494 Schaan (LI)
(72) Erfinder: Bolis, Carlo, 7000, Chur (CH); Grimm, Retro, 7320, Sargans (CH); Huwig, Alexander, 8810, Horgen (CH); Rheinberger, Volker, 9490, Vaduz (LI)
(74) Vertreter: UEXKÜLL & STOLBERG

(56) Entgegenhaltungen:
- WO-A-01/68045
- US-A- 5 425 953
- US-A- 6 083 421
- FONTAINE E. A., TAYLOR-ROBINSON D.: "Comparison of quantitative and qualitative methods of detecting hydrogen peroxide produced by human vaginal strains of lactobacilli" JOURNAL OF APPLIED BACTERIOLOGY, Bd. 69, Nr. 3, 1990, Seiten 326-331, XP008046057

## Beschreibung

Die Erfindung betrifft Zusammensetzungen zum Aufhellen von Zähnen, die als aktiven Bestandteil ein pharmazeutisch annehmbares Oxidationsmittel enthalten.

Zahlreiche Nahrungs- und Genußmittel, wie Tee, Kaffee, Rotwein und Tabak, können zu Verfärbungen der Zähne führen. Auch bestimmte Arzneimittel, wie zum Beispiel Tetrazyklin oder die Aufnahme von großen Mengen an Fluorid können Verfärbungen der Zähne hervorrufen. Da der Mund als Blickfang im Zentrum des Gesichts in einem hohen Maß das äußere Erscheinungsbild bestimmt, werden derartige Verfärbungen oft als eine Beeinträchtigung empfunden und Mittel und Verfahren zu deren Beseitigung finden zunehmendes Interesse.

Ein häufig angewendetes Verfahren umfaßt das Bleichen der Zähne mit konzentrierter Wasserstoffperoxidlösung (30 bis 37 %). Zur Förderung der oxidativen Wirkung des Peroxids werden Hitze und Licht angewendet. Dieses Verfahren führt zwar relativ schnell zum Erfolg, erfordert jedoch einen hohen Aufwand und kann nur durch geschultes Personal durchgeführt werden.

Die US 4,032,627 offenbart pigmenthaltige Lacke zum Überdecken von Verfärbungen. Diese Lacke weisen eine geringe mechanische Belastbarkeit auf und müssen praktisch täglich neu aufgetragen werden.

Die US 5,290,566 offenbart gelförmige Mittel zum Aufhellen von Zähnen, die als aktiven Bestandteil 22 bis 32 Gew.-% Carbamidperoxid enthalten. Diese Gele werden auf eine Schiene aufgebracht, die dann auf die zu behandelnden Zähne aufgesetzt wird. Diese Schiene muß täglich mindestens 6 Stunden getragen werden, bis der gewünschte Aufhellungsgrad erzielt worden ist.

Aus der US 5,171,564 sind pastenförmige Zusammensetzungen bekannt, die neben Carbamidperoxid Schleifmittel enthalten.

Die US 5,425,953 offenbart flüssige Polymerzusammensetzungen, die neben einem Oxidationsmittel wie Carbamidperoxid ein wasserlösliches Cellulosepolymer und vorzugsweise auch einen Stabilisator wie Calciumdinatriumedetat enthalten. Die Zusammensetzungen werden ein- bis dreimal täglich auf die Zähne aufgebracht und bilden dort nach dem Verdampfen des Lösungsmittels einen Film, der das Oxidationsmittel freisetzt und sich innerhalb von etwa 1 Stunde zersetzt.

Schließlich sind aus der US 6,083,421 lackförmige Bleichmittel auf der Basis von Carbamidperoxid bekannt, die für mehr als 1 Stunde an der Zahnoberfläche haften sollen. Bevorzugte Filmbildner sind Polyvinylbutyral, Cumaronharz und Schellack.

Lackförmige Bleichmittel sind in der Regel gut zu handhaben, und ihre Anwendung ist für den Patienten mit relativ wenigen Unannehmlichkeiten verbunden. Nachteilig an bekannten Bleichlacken ist jedoch ihre häufig nur geringe Bleichwirkung, ein geringes Haftvermögen und Feuchtigkeitsempfindlichkeit, so daß Bedarf an weiter verbesserten, filmbildenden Bleichmitteln für Zähne besteht.

Erfindungsgemäß wird diese Aufgabe durch orale Zusammensetzungen gelöst, die
(i) 70 bis 90 Gew.-% Lösungsmittel,
(ii) 2 bis 20 Gew.-% pharmazeutisch annehmbares Oxidationsmittel und
(iii)8 bis 12 Gew.-% Alkylcellulose enthalten.

Gemäß einer Ausführungsform enthalten die Zusammensetzungen neben den genannten Komponenten zusätzlich eine pharmazeutisch annehmbare Säure, vorzugsweise Phosphorsäure (H₃PO₄), Pyrophosphorsäure (H₄P₂O₇) und insbesondere Zitronensäure. Die Säure wird vorzugsweise in einer Menge 0,05 bis 5,0 Gew.-%, insbesondere 0,5 bis 5 Gew.-%, besonders bevorzugt 0,3 bis 0,6 Gew.-% eingesetzt. Alle Prozentangaben beziehen sich, wenn nicht anders angegeben, auf die Gesamtmasse der Zusammensetzung.

Es wurde gefunden, daß geringe Mengen an Säure die Zahnoberfläche konditionieren, d. h. Plaque-, Hydroxylapatit- und Zahnputzmittelreste anlösen und es dem Lack erleichtern, derartige Rückstände zu unterwandern. Durch die Säure wird so eine feste und gleichmäßige Haftung des Lacks an der Zahnoberfläche sichergestellt, ohne jedoch den Zahn zu schädigen. Gleichzeitig wird eine gleichmäßige Benetzung der Zahnoberfläche erzielt, was neben einer guten Haftung ein gleichmäßiges Aufhellen der Zähne gewährleistet. Eine ungleichmäßige Benetzung der Oberfläche oder ein partielles Ablösen eines schlecht haftenden Lacks hat zur Folge, daß die Peroxidverbindung unterschiedlich lang auf verschiedenen Bereiche der Zahnoberfläche einwirkt, was den Zähnen ein unerwünscht fleckiges Aussehen verleihen kann.

Als weitere optionale Komponente enthalten die erfindungsgemäßen Zusammensetzungen vorzugsweise auch Dexpanthenol, vorzugsweise in einer Menge von 0,1 bis 1,0 Gew.-%, besonders bevorzugt 0,2 bis 0,5 Gew.-%. Dexpanthenol ist ein Provitamin, das Irritationen und Reizungen der Mundschleimhaut entgegenwirkt, die durch die Peroxidverbindung, das Lösungsmittel oder die Säure ausgelöst werden können. Darüber hinaus fördert Dexpanthenol die Heilung bereits bestehenden Wunden. Erfindungsgemäße Zusammensetzungen mit einem Gehalt an Dexpanthenol eignen sich daher auch zur Aufhellung von Zähnen bei bestehender Zahnfleischentzündung und sind besser verträglich als herkömmliche Mittel.

Darüber hinaus können der Zusammensetzung weitere Komponenten, wie Aromastoffe, Geschmacksstoffe und/oder Süßstoffe zugesetzt werden. Diese Komponenten werden vorzugsweise in einer Menge von jeweils 0,01 bis 0,5 Gew.-%, besonders bevorzugt 0,02 bis 0,2 Gew.-% und ganz besonders bevorzugt 0,03 bis 0,06 Gew.% verwendet.

Bevorzugte Aroma- und Geschmacksstoffe sind Methylsalicylat, Fenchelholzöl, Nelkenöl, Salbeiöl, Eukalyptusöl, Majoranöl, Menthol und vorzugsweise Minzöl, d.h. Pfefferminz- und/oder Krauseminzöl. Besonders bevorzugt sind synthetische Minzöle und -aromen, insbesondere die von der Firma Haarmann & Reimer unter der Bezeichnung OPTAMINT® vertriebenen Öle und Aromen. Weitere geeignete Aromen sind Erdbeer-, Himbeer-, Bananen-, Kirsch-, Caramel-, Birnen-, Apfel-, Zitronen- und Pfirsicharoma sowie Orangenöl und Grapefruitaroma.

Als Süßstoffe eignen sich besonders Sodiumsaccharin, Sodiumzyklamat, Xylitol, Aspartam und dergleichen. Natriumsaccharin ist besonders bevorzugt.

Bevorzugte pharmazeutisch annehmebare Oxidationsmittel sind Natriumperborat, Chlordioxid und pharmazeutisch annehmbare Peroxidverbindungen, wie z.B. organische Peroxide, vorzugsweise Methylperoxid, Ethylperoxid und insbesondere Glycerinperoxid oder Benzylperoxid. Besonders bevorzugte Peroxide sind Wasserstoffperoxid und insbesondere Carbamidperoxid. Carbamidperoxid wird vorzugsweise in einer Menge von 4 bis 20 Gew.-%, besonders bevorzugt 5 bis 10 Gew.-% und ganz besonders bevorzugte in einer Menge von 5,3 bis 6,5 Gew.-% verwendet. Wasserstoffperoxid wird vorzugsweise in einer Menge von 2 bis 16 Gew.-%, besonders bevorzugt 5,5 bis 13 Gew.-% und am meisten bevorzugt 5,5 bis 12 Gew.-% eingesetzt, gemessen als H₂O₂.

Wasserstoffperoxid wird vorzugsweise in Form einer kommerziell erhältlichen Wasserstoffperoxid-Lösung in Wasser mit einer H₂O₂-Konzentration von 34,5-36,5% eingesetzt. Für höhere Wasserstoffperoxidkonzentrationen wird vorzugsweise eine ebenfalls kommerziell erhältliche wäßrige H₂O₂-Lösung mit einer Konzentration von 48,8-50,6% verwendet.

Das am meisten bevorzugte Oxidationsmittel ist Carbamidperoxid, das in Gegenwart von Wasser zu Harnstoff und Wasserstoffperoxid hydrolysiert. Der gebildete Harnstoff hat den Vorteil, daß er pH-neutralisierend wirkt. Das freigesetzte Wasserstoffperoxid diffundiert in den Zahnschmelz und das Dentin, wo es bei der Oxidation zu Wasser und Sauerstoff zerfällt. Durch die Diffusion des Wasserstoffperoxids in den Zahnschmelz wird das chemische Gleichgewicht zwischen Carbamidperoxid und seinen Zerfallsprodukten gestört, was die weitere Freisetzung von Wasserstoffperoxid aus dem Carbamidperoxid zur Folge hat.

Durch die erfindungsgemäßen Lacke wird sichergestellt, daß das Wasserstoffperoxid praktisch vollständig in den Zahnschmelz diffundiert und nicht wie bei herkömmlichen Lacken zu großen Teilen in Speichel und Weichgewebe abgegeben wird.

Ein wesentlicher Aspekt der Erfindung besteht in der Verwendung von Alkylcellulose als Lackbildner. Vorzugsweise wird eine wasserunlösliche Alkylcellulose verwendet, d.h. Alkylcellulose mit einer Löslichkeit von maximal 0,1 g Alkylcellulose pro 100 g Wasser bei Raumtemperatur. Besonders bevorzugt ist Ethylcellulose, insbesondere Ethylcellulose mit einem mittleren Substitutionsgrad (DS) von > 1,5, besonders bevorzugt > 2,0, insbesondere 2,0 bis 2,6, und ganz besonders bevorzugt 2,3 bis 2,4. In Cellulose weist jede Glucoseeinheit drei freie Hydroxylgruppen auf. Sind alle drei Hydroxylgruppen aller Glucoseeinheiten der Cellulose durch Alkoxygruppen substituiert, beträgt der Substitutionsgrad (DS) 3. Ist nur ein Teil der Hydroxylgruppen der Glucoseeinheiten mit Alkoxygruppen substituiert, ist der Substitutionsgrad entsprechend geringer. Der Substitutionsgrad DS gibt die durchschnittliche Anzahl an substituierten Hydroxylgruppen pro verknüpfter Glucoseeinheit an. Ganz besonders bevorzugt ist Ethylcellulose, die einen Ethoxylgehalt von 45 bis 50 % aufweist. Die Alkylcellulose wird vorzugsweise in einer Menge von 9 bis 10 Gew.-% eingesetzt.

Die Alkylcellulose weist weiter bevorzugt eine Viskosität von 50 bis 150 mPas, besonders bevorzugt 80 bis 110 mPas, ganz besonders bevorzugt 90 bis 100 mPas auf, gemessen bei 25 °C mit einem Ubbelohde-Viskosimeter an einer 5 Gew.-%igen Lösung in Toluol/Ethanol 80:20 (v/v).

Die Verwendung von Alkylcellulose und insbesondere von Ethylcellulose als Lackbildner verleiht den erfindungsgemäßen Zusammensetzungen eine gewisse Wassertoleranz, d.h. die Wirkung der Zusammensetzung wird durch den Kontakt beispielsweise mit Speichel nicht beeinträchtigt. Sollte der Lack nach dem Auftragen auf den Zahn mit Speichel in Berührung kommen, was bei Anwendung durch den Patienten selbst meist nicht auszuschließen ist, wird der Lack nicht abgespült, sondern es kommt zu einer Ausfällung des Lackbildners, wodurch eine Schutzschicht ausgebildet wird, welche die Lackschicht vor einem Eindringen des Speichels und einem Ablösen vom Zahn schützt. Gleichzeitig wird ein Auswaschen des Oxidationsmittels durch den Speichel verhindert und sichergestellt, daß das Oxidationsmittel nach dem Aufbringen der Zusammensetzung in den Zahnschmelz diffundiert. Dies ist insbesondere bei der Verwendung wasserlöslicher Peroxidverbindungen ein wesentlicher Vorteil. Die erfindungsgemäßen Zusammensetzungen gewährleisten so bei relativ geringer Konzentrationen an Oxidationsmittel und geringer Behandlungszeit eine deutliche Aufhellung der Zähne. Im Gegensatz dazu beobachtet man bei herkömmlichen Bleichmitteln häufig ein rasches Auswaschen wasserlöslicher Peroxidverbindungen, so daß eine im Verhältnis zur Oxidationsmittelkonzentration nur geringe Bleichwirkung erzielt wird. Eine geringe Konzentration der häufig aggressiven Bleichmittel ist bei Mitteln, die zur Anwendung durch den Patienten selbst gedacht sind, im Hinblick auf die Anwendungssicherheit und mögliche Nebenwirkungen ein großer Vorteil.

Bevorzugte Lösungsmittel sind Diethylehter, Aceton, vorzugsweise Ethylacetat und insbesondere Ethanol. Mischungen aus einem oder mehreren dieser Lösungsmittel können verwendet werden. Darüber hinaus kann das Lösungsmittel bis zu 30 Gew.-% an Wasser enthalten, bezogen auf die Gesamtlösungsmittelmenge, d.h. auf die Menge an Wasser und weiterem Lösungsmittel. Bei der Verwendung von Carbamidperoxid liegt der Wassergehalt vorzugsweise unter 12 Gew.-%, besonders bevorzugt unter 2 Gew.-%, wobei wasserfreie Zusammensetzungen am meisten bevorzugt sind.

Bei den erfindungsgemäßen Zusammensetzungen handelt es sich um farblose bis weiße Lacke, die gemäß einer bevorzugten Ausführungsform neutral sind, d.h. einen pH-Wert im Bereich von 5,5 bis 8, vorzugsweise 6,5 bis 7,5 aufweisen, gemessen an einer wäßrigen Dispersion der Zusammensetzung (1 bis 25 Gew.-%). Die Lacke tropfen nicht, ziehen beim Auftragen keine Fäden und lassen sich mit einem Pinsel präzise auftrage. Sie trocknen innerhalb von etwa 1 Minute.

Säurehaltige Zusammensetzungen weisen vorzugsweise einen pH-Wert von 1 bis 6, besonders bevorzugt 2 bis 4 und ganz besonders bevorzugt 2 bis 3 auf.

Die Zusammensetzungen können gemäß einer Ausführungsform auch Farbstoffe enthalten, die keinen negativen Einfluß auf die Bleichwirkung haben, beispielsweise Carmin und Carthamin. Durch das Verwenden gefärbter Zusammensetzungen läßt sich leichter sicherstellen, daß die Zahnoberfläche vollständig benetzt ist. Die erfindungsgemäßen Zusammensetzungen enthalten vorzugsweise keine Schleifmittel.

Erfindungsgemäß bevorzugte Zusammensetzungen enthalten die folgenden Komponenten:
(i) 70,0 bis 90,0 Gew.-%, insbesondere, 80 bis 88 Gew.-%, ganz besonders 84 bis 86 Gew.-% Lösungsmittel,
(ii) 2 bis 16 Gew.-% Oxidationsmittel, insbesondere 5,3 bis 6,5 Gew.-% Carbamidperoxid oder 5,5 bis 13 Gew.-% Wasserstoffperoxid,
(iii)8,0 bis 12,0 Gew.-%, insbesondere 9 bis 10 Gew.% Alkylcellulose, vorzugsweise Ethylcellulose,
(iv) 0 bis 5,0 Gew.-%, insbesondere 0,5 bis 5 Gew.-%, besonders bevorzugt 0,3 bis 0,6 Gew.-% Säure, vorzugsweise Zitronensäure,
(v) 0 bis 1,0 Gew.-%, insbesondere 0,2 bis 0,5 Gew.-% Dexpanthenol,
(vi) 0 bis 0,5 Gew.-%, insbesondere 0 bis 0,2 Gew.-%, besonders bevorzugt 0,01 bis 0,05 Gew.-% Geschmacks- und/oder Aromastoff, insbesondere Minzöl oder Minzaroma.

Ein besonders bevorzugtes Bleichmittel auf der Basis von Wasserstoffperoxid weist folgende Zusammensetzung auf:
(i) 55 bis 72 Gew.-% Ethanol,
(ii) 5,5 bis 13,0 Gew.-% Wasserstoffperoxid,
(iii)8,5 bis 9,5 Gew.-% Ethylcellulose,
(iv) 15 bis 28 Gew.-% Wasser,
(v) 0,2 bis 0,5 Gew.-% Dexpanthenol und
(vi) 0,01 bis 0,06 Gew.-% Aroma.

Der Wassergehalt bezieht sich hier auf die Gesamtmasse der Zusammensetzung, wobei jedoch zusätzlich auch der oben definierte maximale Wassergehalt bezogen auf die Gesamtlösungsmittelmenge zu beachten ist.

Ein ganz besonders bevorzugtes Bleichmittel auf der Basis von Carbamidperoxid weist folgende Zusammensetzung auf:
(i) 79 bis 85 Gew.-% Ethanol,
(ii) 5,3 bis 6,5 Gew.-% Carbamidperoxid,
(iii)8,5 bis 9,5 Gew.-% Ethylcellulose,
(iv) 0 oder 0,3 bis 0,6 Gew.-% Zitronensäure,
(v) 0,2 bis 0,5 Gew.-% Dexpanthenol und
(vi) 0,01 bis 0,06 Gew.-% Aroma.

Die erfindungsgemäßen Zusammensetzungen werden hergestellt, indem die Komponenten in den gewünschten Mengen miteinander gemischt werden, bis eine homogene, streichfähige Lösung erhalten wird.

Zur Anwendung werden die zu behandelnden Zähne zunächst gründlich gereinigt und getrocknet, z.B. durch Anblasen mit einem Luftstrahl oder durch Abtupfen mit einem Zellstofftuch. Anschließend wird die erfindungsgemäße Zusammensetzung mit einem Applikator, z.B. einem kleinen Pinsel auf die Zahnoberfläche aufgebracht und trocknen gelassen. Das Trocknen kann durch das Anblasen mit einem Luftstrahl beschleunigt werden. Beim Verdampfen des Lösungsmittels bildet sich ein farbloser bis weißer oder ggf. gefärbter Film, der fest an der Zahnoberfläche haftet und vorzugsweise über einen Zeitraum von 15 bis 30 Minuten im Mund verbleibt. Während dieser Phase sollte der Patient möglichst wenig sprechen und keine flüssige oder feste Nahrung zu sich nehmen. Nach Ablauf der Einwirkzeit läßt sich der Lack wie ein Film abziehen oder mit einer nassen Zahnbürste (ohne Zahnpasta) entfernen. Die Gesamtanwendungsdauer und -häufigkeit richtet sich nach dem gewünschten Bleichergebnis. Empfehlenswert ist eine zweimal tägliche Anwendung über einen Zeitraum von vierzehn Tagen. Sollte das gewünschte Ergebnis bereits vor Ablauf dieses Zeitraumes erzielt werden, kann die Behandlung abgebrochen oder anderenfalls verlängert werden.

Speichel enthält das Enzym Katalase, das die Disproportionierung von Wasserstoffperoxid unter Bildung von Wasser und Sauerstoff katalysiert. Katalase kann damit eine Zersetzung des in den Bleichmitteln enthaltenen Wasserstoffperoxids bewirken, ohne daß dieses in den Zahnschmelz eindringen und dort Farbstoffe bleichen könnte. Katalase kann auch dann problematisch sein, wenn Speichel beispielsweise mit dem Pinsel in das Bleichmittelgefäß eingeschleppt wird und dort eine Zersetzung des Oxidationsmittels bewirkt. Es wurde gefunden, daß erfindungsgemäße Zusammensetzungen auf der Basis von Carbamidperoxid selbst nach Zusatz von 8 Vol.-% Speichel über einen Zeitraum von 1 Woche keine nennenswerte Veränderung der Carbamidperoxidkonzentration zeigen, was auf eine bemerkenswerte Unempfindlichkeit der erfindungsgemäßen Zusammensetzungen gegenüber Speichel hindeutet.

Aus Hygienegründen werden die erfindungsgemäßen Zusammensetzungen dennoch vorzugsweise in solchen Verpackungen zur Verfügung gestellt, bei denen der Applikator nicht mit dem Inhalt selbst in Kontakt kommt. Dies ist auch im Hinblick auf eine mögliche Präzipitation des Lackbildners bei Speichelkontakt vorteilhaft. Eine bevorzugte Verpackungseinheit enthält ein Gefäß, vorzugsweise eine Tube, mit der erfindungsgemäßen Zusammensetzung, ein Gefäß, vorzugsweise eine kleine Schale, zur Aufnahme der zu einer einmaligen Behandlung der Zähne erforderlichen Menge an Bleichmittel und einen Applikator wie beispielsweise einen Pinsel. Zur Anwendung der Zusammensetzung wird die benötigte Menge an Bleichmittel von der Tube in die Schale überführt und dann mit dem Applikator auf die Zähne aufgebracht. Auf diese Weise wird ein Kontakt der in der Tube befindlichen Zusammensetzung mit dem benutzten Applikator vermieden.

Ein besonderes Problem bei der Bestimmung der Wirksamkeit von Bleichmitteln für Zähne ist, daß hierzu in der Regel aufwendige Untersuchungen an extrahierten menschlichen Zähnen oder Rinderzähnen erforderlich sind. Erfindungsgemäß wurde ein Testsystem entwickelt, daß auf einfache Weise ein Abschätzen der Bleichwirkung ermöglicht. Verfärbungen der Zähne sind häufig auf aromatische Verbindungen zurückzuführen, die beim Bleichen in farblose, gesättigte Kohlenwasserstoffe überführt werden. Es ist jedoch bekannt, daß bestimmte aromatische Verbindungen, wie zum Beispiel der Farbstoff o-Dianisidin, bei der Oxidation durch Wasserstoffperoxid gefärbte Produkte bilden. o-Dianisidin bildet ein stabiles, dunkelbraun gefärbtes Kation, das sich visuell nachweisen läßt und so einen Nachweis der oxidativen Wirkung von Peroxiden ermöglicht. Der erfindungsgemäße Test nutzt diese Reaktion zur Abschätzung der Wirkung von Bleichmitteln aus.

Zur Durchführung des Tests wird eine aromatische Verbindung, die bei der Oxidation durch Wasserstoffperoxid farbige Produkte bildet, in eine gelierfähige Lösung eingebracht und gelieren gelassen. Das zu untersuchende Bleichmittel wird in einer bestimmten Menge auf einen Prüfkörper, vorzugsweise aus Porzellan, Glas, Keramik, Glaskeramik und besonders bevorzugt aus einer dentalen Glaskeramik aufgebracht und trockenen gelassen. Besonders geeignet sind zylinderförmige Prüfkörper, beispielsweise mit einem Durchmesser von etwa 1 cm. Diese beschichteten Testkörper werden anschließend auf das Gel aufgelegt, wobei die beschichtete Seite zum Gel weist. In dem Bleichmittel enthaltenes Peroxid diffundiert in das Gel und reagiert dort mit der aromatischen Verbindung unter Ausbildung eines kreisförmigen, gefärbten Hofes um die Testkörper herum. Der Durchmesser des Hofes ist proportional zur Menge an Peroxid, das aus dem Bleichmittel freigesetzt wird. Der Test erlaubt eine Abschätzung der Geschwindigkeit und der Menge der Wasserstoffperoxidfreisetzung. Er ist geeignet zur Untersuchung von Zusammensetzungen, die als oxidierende Komponente Wasserstoffperoxid, Carbamidperoxid, Methylperoxid oder Ethylperoxid enthalten.

Als aromatische Verbindung eignen sich besonders Verbindungen aus der Gruppe der Phenole, Aminophemole, aromatischen Diamine, Indophenole und Leucofarbstoffe. Ganz besonders bevorzugt ist o-Dianisidin. o-Dianisidin weist eine bräunliche Färbung auf, die bei der Oxidation durch Peroxid in ein dunkles braun übergeht. Da die Reaktion von Peroxid mit o-Dianisidin nur relativ langsam verläuft, wird dem Agar vorzugsweise auch das Enzym Peroxidase zugegeben, das diese Reaktion katalysiert. Eine andere bevorzugte aromatische Verbindung ist 2,2'-Azinobis(3-ethylbenzthiazolin-6-sulfonsäure (ABTS).

Eine besonders bevorzugte gelierende Lösung weist die folgende Zusammensetzung auf:
(a) 1 bis 30 Gew.-%, vorzugsweise 5 bis 25 Gew.-% und besonders bevorzugt 10 bis 20 Gew.-% Gelbildner;
(b) 0,01 bis 0,1 Gew.-%, vorzugsweise 0,015 bis 0,05 Gew.-% und besonders bevorzugt 0,02 bis 0,03 Gew.-% aromatische Verbindung, vorzugsweise o-Dianisidin;
(c) ggf. 0,001 bis 0,01 Gew.-%, vorzugsweise 0,0015 bis 0,003 Gew.-% Peroxidase;
(d) Pufferlösung, vorzugsweise NaK-Phosphatpuffer (ad 100 Gew.-%).

Die Agarlösung wird vorzugsweise in Petrischalen gegossen und gelieren gelassen.

Bevorzugte Gelbildner sind Cellulosederivate, Stärkederivate, Polysaccharide, Gelatine und Kieselgel, insbesondere Agarose, anionische, saure Heteropolysaccharide, wie Gellan, und insbesondere Agar-Agar.

Im folgenden wird die Erfindung anhand von Ausführungsbeispielen im Zusammenhang mit den Figuren näher erläutert, wobei:
Figur 1 eine rasterelektronenmikroskopische Aufnahme eines erfindungsgemäßen Lacks nach dem Trocknen zeigt, der auf einen Glasobjekträger aufgetragen ist.
Figur 2 eine graphische Darstellung des zeitlichen Verlaufs der Carbamidperxidfreisetzung aus einem erfindungsgemäßen Lacks zeigt.

### Ausführungsbeispiele

### Beispiel 1: Herstellung eines Bleichmittels auf der Basis von Carbamidperoxid

108 ml wasserfreies Ethanol wurden in ein Becherglas gegeben und mit 9 g Ethylcellulose versetzt. Die Mischung wurde solange gerührt, bis eine homogene Lösung vorlag. Diese wurde nacheinander mit 5,9 g reines Carbamidperoxid, 0,3 g Dexpanthenol und 0,1 g Minzaroma (OPTAMINT®, Haarmann & Reimer) versetzt und gleichmäßig verrührt. Es wurde ein farbloser bis weißer Lack erhalten.

### Chemische und physikalische Daten des Lacks:

| | |
|---|---|
| Aussehen : | viskose, opake, farblose Flüssigkeit |
| Geruch : | Pfefferminze |
| Viskosität (bei 100 s⁻¹) : | 2.50 - 4.00 Pa*s |
| Dichte : | 0.83-0.84 g / ml |
| pH-Wert | |
| (1-25% in Wasser dispergiert) : | 6.5 - 7.5 |
| Flammpunkt : | 13 °C |

### Beispiel 2: Lagerstabilität des Lacks aus Beispiel 1

Um die Lagerstabilität des Lacks aus Beispiel 1 zu ermitteln wurde in einer 100 ml Glasflasche bei Raumtemperatur für einen Zeitraum von 52 Wochen aufbewahrt. Zu Beginn und am Ende des Untersuchungszeitraumes wurde der Gehalt des Oxidationsmittels (Carbamidperoxidgehalt) titrimetrisch ermittelt. Nach 52 Wochen wurde ein Carbamidperoxidgehalt von 5,12 % gemessen, die titrimetrisch ermittelte Ausgangskonzentration betrug 6,23 %. Die Bestimmung erfolgte gemäß U.R. Kunz (1990): Grundlagen der Quantitativen Analyse, Thieme-Verlag, Stuttgart, 3. Auflage. Der Lack erwies sich damit als ausreichend lagerstabil.

### Beispiel 3: Ermittlung der Schichtdicke des Lacks nach dem Trocknen

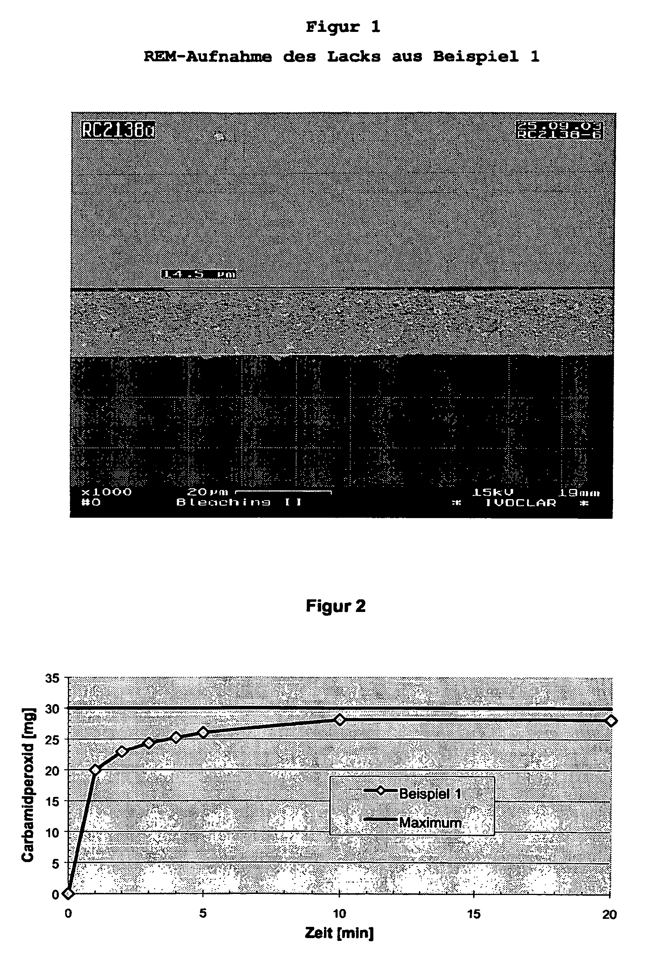

Der Lack aus Beispiel 1 wurde mit einem Pinsel gleichmäßig in einer Schicht auf einen Objekträger aus Glas aufgetragen und trocknen gelassen. Nach dem Trocknen wurde die Lackschicht elektronenmikroskopisch untersucht. Es wurde eine Schichtdicke von 10 bis 20 µm gemessen (Figur 1). Bei 1000facher Vergrößerung ist zu erkennen, daß der Lack eine sehr gleichmäßige, geschlossene Schicht mit klar strukturierter Textur bildet.

### Beispiel 4: Herstellen einer Agar-Lösung zur Bestimmung der Bleichwirkung

Um den Prozeß des Zähnebleichens simulieren zu können, wurde ein enzymatisch basierter Agar-Test etabliert, mit dem die Freisetzung des Wasserstoffperoxids verfolgt werden kann.

Die Herstellung des Agars erfolgte folgendermaßen:
1. Einrühren von 50 mg o-Dianisidin und 3 g Agar-Agar in 190 ml 50 mM NaK-Phosphatpuffer, pH7; zur optimierten Verteilung des o-Dianisidins erfolgte das Mischen unter Einsatz eines Ultraschallbades.
2. 10minütiges Autoklavieren bei 100°C der mit einem Deckel leicht verschlossenen Flasche.
3. Nach dem Abkühlen auf 50°C Zugabe von 3 mg Peroxidase unter Rühren, welche in 10 ml Puffer vorgelöst und unter Verwendung einer Luer-Lock-Spritze durch einen Sterilfilter zugegeben wurde.
4. Gießen des Agars in Petrischalen (Durchmesser 9 cm und 15 cm). Es ist auf eine gleichmässige Befüllung der Petrischalen zu achten. Die Füllmenge betrug bei Petrischalen von 9 cm Durchmesser 15 g, bei Petrischalen von 15 cm Durchmesser 40 g.

Die Agarplatten können kopfüber bei 8°C bis zu zwei Wochen gelagert werden.

Von dem zu testenden Bleichmittel (Bleichlack aus Beispiel 1) wurden genau 15 mg auf einen Prüfkörper aus Dentalkeramik (d=1cm, Tetric Flow, Firma Ivoclar Vivadent AG) aufgetragen, trocknen gelassen und der Prüfkörper mit der beschichteten Seite auf die Agaroberfläche gelegt. Es erfolgte eine Freisetzung von Carbamidperoxid mit anschließender Hydrolyse zu Wasserstoffperoxid und Harnstoff in dem hauptsächlich aus Wasser bestehenden Agar. Das freigesetzte Wasserstoffperoxid bewirkt eine Braunfärbung des Agars, die so lange unter Verwendung einer Schieblehre ausgemessen wurde, bis die Kreisgröße rückläufig war.

Die Braunfärbung beruht darauf, daß im Agar Elektronen von dem Chromogen o-Dianisidin auf Waserstoffperoxid übertragen werden, das zu Wasser reduziert wird. Das oxidierte Chromogen bildet einen stabilen Farblack, dessen Entstehung proportional zur aufgetragenen Wasserstoffperoxidmenge ist und der aufgrund seiner intensiv braunen Farbe sehr gut erkannt werden kann. Wasserstoffperoxid oxidiert o-Dianisidin allerdings nur sehr langsam, so daß dem Agar zudem das Enzym Peroxidase als Biokatalysator hinzugegeben wurde. Der Agar wird hierbei zur Verfestigung des flüssigen Testmediums eingesetzt, um die Freisetzung gezielt verfolgen zu können.

Der Test erlaubt eine reihenmäßige Untersuchung von Bleichmitteln innerhalb von Stunden.

### Beispiel 5: Quantitative Bestimmung der Peroxidfreisetzung

Um die Freisetzung des Carbamidperoxids aus dem Lack zu messen, wurde ein mit der Zusammensetzung aus Beispiel 1 beschichteter Probekörper in eine Petrischale gegeben und mit Wasser überschichtet. Nach verschiedenen Zeitintervallen wurden Proben genommen und die Konzentration des entsprechenden Peroxids mit einem enzymatischen Test bestimmt. In diesem Test wird Peroxidase als Katalysator und 2,2'-Azinobis(3-ethylbenzthiazolin-6-sulfonsäure) (ABTS) als Chromogen verwendet (Werner W., Rey H.G., Wielinger H. (1970): Über die Eigenschaften eines neuen Chromogens für die Blutzuckerbestimmung nach der GOD/POD-Methode, Z. Anal. Chem. 252, 224-228; Michal G., Möllering H., Siedel J. (1983): Chemical design of indicator reactions for the visible range, in: Bergmeyer H.U. (Herausgeber), Methods of enzymatic analysis, Vol. 1, Verlag Chemie, Weinheim, 197-232). In dem photometrischen Test oxidiert Wasserstoffperoxid in einer nachfolgenden, durch Peroxidase katalysierten Indikatorreaktion das Chromogen ABTS zu einem intensiv blaugrünen Farbstoff, dessen Konzentration durch direkte photometrische Messung ermittelt wurde und dessen Konzentration der Wasserstoffperoxidkonzentration proportional war. Die Messung erfolgte bei 420 nm, der Extinktionskoeffizient von ABTS⁺ beträgt bei 420 nm 43,2 ml µmol⁻¹ cm⁻¹.

ABTS ist ein heterozyklisches Azin, das mit zwei höheren Oxidationsstufen ein durch zwei Ein-Elektronenübergänge verbundenes, reversibles Redoxsystem darstellt. Im ersten Oxidationsschritt wird das farblose bis leicht hellgrüne Azin zu einem blaugrünen gefärbten Radikalkation (ABTS⁺) oxidiert. Der zweite Oxidationsschritt vom Radikalkation zum dunkel-violetten Azodikation tritt nur bei großem Überschuß des Oxidationsmittels und in stark saurem Milieu auf.

### Im Einzelnen wurde die Messung wie folgt durchgeführt:

Eine Standardkurve wurde aufgenommen, indem Lösungen mit vier verschiedenen Konzentrationen an Wasserstoffperoxid hergestellt wurden: 0.010 mg/ml, 0.013 mg/ml, 0.020 mg/ml und 0.025 mg/ml. Diese Konzentrationen führten zu Extinktionswerten im Bereich von 0.1 - 0.7, dem linearen Bereich, in dem die Zunahme der Extinktion direkt proportional der Reduktion von HP zu Wasser ist.

960 µl ABTS Lösung (2.08 mM ABTS in 50 mM Kaliumphosphatpuffer, pH 6.0) und 20 µl Peroxidase (400 U/ml in 50 mM Kaliumphosphatpuffer, pH 6.0) wurden in eine Küvette gegeben, in den Probenhalter des Photometers gestellt und während 10 s gemessen. Danach wurde die zu messende Peroxidlösung hinzugegeben, zur Durchmischung kurz geschüttelt und wieder gemessen. Die Extinktion wurde über 2 Minuten verfolgt.

Die gemessenen Mengen an freigesetztem Peroxid werden kumuliert graphisch gegen die Zeit aufgetragen (Figur 2). Es wurde gefunden, daß mehr als 80 % des Peroxides innerhalb der ersten 5 Minuten freigesetzt wurden, 90 % innerhalb der ersten 10 Minuten. Nach 15 Minuten war das Carbamidperoxid praktisch vollständig aus dem Lack herausgelöst.

Diese Methode ist insbesondere für die schnelle Bestimmung der Wasserstoffperoxidkonzentration sowie für Freisetzungsuntersuchungen sehr gut geeignet.

### Beispiel 6: Bestimmung der Katalaseempfindlichkeit

Um die Empfindlichkeit erfindungsgemäßer Zusammensetzungen gegenüber Katalase zu bestimmen, wurde der Lack aus Beispiel 1 mit 2,5 Vol.-% bzw. 8 Vol.-% Speichel versetzt und 10 Tage bei Raumtemperatur inkubiert. Der Speichel war mit einer physiologisch signifikanten Menge Katalase versetzt. Es wurde gefunden, daß die Carbamidperoxidkonzentration durch den Zusatz von 2.5 Vol% Speichel innerhalb des Untersuchungszeitraumes konstant blieb. In der mit 8 Vol.-% Speichel versetzten Probe blieb die Carbamidperoxidkonzentration für etwa 7 Tage unverändert.

Um zu überprüfen, ob mit der eingesetzten Methode eine Katalase-Aktivität im Speichel und ein Abbau von Carbamidperoxid festgestellt werden kann, wurden weitere Tests durchgeführt, bei denen Speichel zusätzlich mit kommerziell erhältlicher Katalase aus *Aspergillus niger* versetzt wurde. Hierbei konnte ein deutlicher Abbau von Carbamidperoxid festgestellt werden.

### Beispiel 7: Nachweis der Bleichwirkung des Lacks aus Beispiel 1

Zur Bestimmung der Bleichwirkung des Lacks aus Beispiel 1 wurde eine interne klinische *in vivo* Studie mit 5 freiwilligen Probanden durchgeführt.

Von allen Probanden wurde eine allgemeine Anamnese aufgenommen und ein zahnärztlicher Befund erstellt. Bei allen Probanden wurde vor Behandlungsbeginn eine professionelle Zahnreinigung durchgeführt. Erst nach der Zahnreinigung wurde die Ausgangssituation dokumentiert und die aktuelle Zahnfarbe anhand der Chromascop-Farbskala bestimmt und fotographisch dokumentiert (frontale Übersichtsaufnahme mit Chromascop-Referenzprobe im Bild).

Vor jeder Lackapplikation wurden alle Zahnoberflächen mit Nylonbürstchen und Prophylaxepaste von weichen Belägen befreit. Es folgte die Trockenlegung der zu behandelnden Zähne (15-25 und 35-34) mit Luftpuster und Speichelsauger. Zur Erleichterung der Erreichbarkeit der zu behandelnden Zähne wurde ein Lippen-Wangenhalter (nach Dr. Horvath bzw. Spandax von Hager & Werken) eingesetzt.

Der Lack wurde mit einem Pinsel gleichmäßig in einer Schicht auf die Zahnoberflächen aufgetragen. Der Lack hatte eine sehr gute Konsistenz und ließ sich mit dem Pinsel präzise auftragen, tropfte nicht und zog keine Fäden. Er bildete eine gleichmäßige Schicht und benetzte auch die Approximalbereiche sehr gut. Nach Abschluß der Applikation wurde der Lippen-Wangen-Halter weitere 60 Sekunden im Munde des Patienten belassen, um eine ausreichende Trocknung des Materials sicherzustellen. Erst nach Ablauf dieser Zeit wurde der Lippen-Wangen-Halter entfernt. Der Patient wurde aufgefordert, für die Dauer der Einwirkzeit die Zähne nicht mit der Zunge zu berühren. Nach 15 Minuten Einwirkzeit wurde das Material mit einem Nylonbürstchen und Wasserspray entfernt und die Schleimhaut auf Irritationen hin untersucht. Dieses Vorgehen wurde einmal täglich solange wiederholt, bis sich der gewünschte Bleicherfolg eingestellt hatte, jedoch nicht länger als über einen Zeitraum von 4 Wochen respektive 20 Behandlungen.

Bei jeder fünften Sitzung erfolgte eine Dokumentation der aktuellen Situation. Hierzu wurde die aktuelle Zahnfarbe entsprechend der Chromascop-Farbscala bestimmt und fotographisch dokumentiert. Nach der letzten Applikation wurde erneut die aktuelle Situation dokumentiert. Die aktuelle Zahnfarbe wurde bestimmt und fotographisch dokumentiert. In weiteren Nachkontrollen wurde evaluiert, ob der Bleicherfolg stabil bleibt.

Der Bleicheffekt wurde frühestens nach einer Zeit von etwa 5 Tagen (5 Anwendungen) festgestellt. Bei dem überwiegenden Teil der Patienten stellte sich eine endgültige Aufhellung nach etwa 10 Anwendungen ein.

Die Aufhellung der Zähne erfolgte sehr gleichmäßig mit leichter Betonung der inzisalen Hälfte. Die Probanden wurden im Durchschnitt etwa 14 mal (Mittel: 13,8 ± 3,9 Anwendungen) mit dem Bleichmittel behandelt. Trotz der durchschnittlich sehr hellen Ausgangsfarbe der Probanden wurde im Mittel deutlich mehr als eine Helligkeitsstufe auf der Chromascopskala gewonnen (Mittel: 1,6 ± 0,5 Helligkeitsstufen). In einen Fall wurde nach 15 Anwendungen eine Aufhellung um zwei Stufen (von 130 nach 110) erzielt. Die Aufhellung blieb über den Untersuchungszeitraum von 6 Monaten stabil.

Die Patienten empfanden den Lack- als geruchs- und geschmackneutral und fühlten fast keine vom Lack ausgehenden störenden Einflüsse.

### Beispiel 8: Herstellung eines Bleichmittels auf der Basis von Wasserstoffperoxid

89 ml wasserfreies Ethanol wurden in ein Becherglas gegeben und mit 9.0 g Ethylcellulose versetzt. Die Mischung wurde solange gerührt, bis eine homogene Lösung vorlag. Diese wurde nacheinander mit 20.0 g Wasserstoffperoxidlösung (30%ig), 0.3 g Dexpanthenol und 0.1g Minzaroma (Optamint® Krauseminzaroma 218921, Haarmann & Reimer) versetzt und gleichmäßig verrührt. Es wurde ein opaker Lack erhalten.

Chemische und physikalische Daten des Lackes:

| | |
|---|---|
| Aussehen : | viskose, opake, farblose Flüssigkeit |
| Geruch : | Pfefferminze |
| Viskosität (bei 100s⁻¹) : | 1.5 - 2.5 Pa*s |
| Dichte | 0.86 - 0.88 g / ml |
| pH-Wert | |
| (1-25% in Wasser dispergiert) : | 5.5 - 6.5 |

## Patentansprüche

1. Orale Zusammensetzung enthaltend:
(i) 70 - 90 Gew.-% Lösungsmittel,
(ii) 2 - 20 Gew.-% pharmazeutisch annehmbares Oxidationsmittel und
(iii) 8 - 12 Gew.-% Alkylcellulose, jeweils bezogen auf die Gesamtmasse der Zusammensetzung.

2. Zusammensetzung nach Anspruch 1, die zusätzlich (iv) pharmazeutisch annehmbare Säure enthält.

3. Zusammensetzung nach Anspruch 2, die zusätzlich Zitronensäure enthält.

4. Zusammensetzung nach Anspruch 2 oder 3, die 0,05 - 5,0 Gew.-% Säure enthält, bezogen auf die Gesamtmasse der Zusammensetzung.

5. Zusammensetzung nach einem der Ansprüche 1 bis 4, die zusätzlich (v) Dexpanthenol enthält.

6. Zusammensetzung nach Anspruch 5, die 0,1 bis 1,0 Gew.-% Dexpanthenol enthält, bezogen auf die Gesamtmasse der Zusammensetzung.

7. Zusammensetzung nach einem der Ansprüche 1 bis 6, die zusätzlich (vi) Aromastoff, Geschmacksstoff und/oder Süßstoff enthält.

8. Zusammensetzung nach einem der Ansprüche 1 bis 7, die als Lösungsmittel Ethanol, Diethylether, Ethylacetat und/oder Aceton enthält.

9. Zusammensetzung nach Anspruch 8, die als Lösungsmittel Ethanol enthalt.

10. Zusammensetzung nach einem der Ansprüche 1 bis 9, die als Alkylcellulose eine Alkylcellulose mit einer Wasserlöslichkeit von maximal 0,1 g in 100 g Wasser bei Raumtemperatur enthält.

11. Zusammensetzung nach einem der Ansprüche 1 bis 10, die Alkylcellulose mit einer Viskosität von 50 bis 150 mPas enthält, gemessen bei 25 °C mit einem Ubbelohde-Viskosimeter an einer 5 Gew.-%igen Lösung in Toluol/Ethanol 80:20 (v/v).

12. Zusammensetzung nach Anspruch 10 oder 11, die als Alkyl-cellulose Ethylcellulose enthält.

13. Zusammensetzung nach einem der Ansprüche 7 bis 12, die als Aromastoff Minzöl enthält.

14. Zusammensetzung nach einem der Ansprüche 1 bis 13, die als pharmazeutisch annehmbares Oxidationsmittel eine Peroxid-verbindung enthält.

15. Zusammensetzung nach einem der Ansprüche 1 bis 14, die
(i) 70, 0 bis 90, 0 Gew.-% Ethanol,
(ii) 2 bis 16,0 Gew.-% Oxidationsmittel,
(iii)8,0 bis 12,0 Gew.-% Ethylcellulose,
(iv) 0 bis 5,0 Gew.-% Zitronensäure,
(v) 0 bis 1,0 Gew.-% Dexpanthenol,
(vi) 0 bis 0,5 Gew.-% Aroma enthält, jeweils bezogen auf die Gesamtmasse der Zusammensetzung.

16. Zusammensetzung nach Anspruch 14 oder 15, die als Peroxidverbindung Carbamidperoxid enthält.

17. Zusammensetzung nach Anspruch 16, die
(i) 79 bis 85 Gew.-% Ethanol,
(ii) 5,3 bis 6,5 Gew.-% Carbamidperoxid,
(iii) 8,5 bis 9,5 Gew.-% Ethylcellulose,
(iv) 0 oder 0,3 bis 0,6 Gew.-% Zitronensäure,
(v) 0,2 bis 0,5 Gew.-% Dexpanthenol und
(vi) 0,01 bis 0,06 Gew.-% Aroma enthält, jeweils bezogen auf die Gesamtmasse der Zusammensetzung.

18. Zusammensetzung nach Anspruch 14 oder 15, die als Peroxidverbindung Wasserstoffperoxid enthält.

19. Zusammensetzung nach Anspruch 18, die bis zu 30 Gew.-% Wasser enthält, bezogen auf die Gesamtlösungsmittelmenge.

20. Zusammensetzung nach Anspruch 18 oder 19, die
(i) 55 bis 72 Gew.-% Ethanol,
(ii) 5,5 bis 13,0 Gew.-% Wasserstoffperoxid,
(iii)8,5 bis 9,5 Gew.-% Ethylcellulose,
(iv) 15 bis 28 Gew.-% Wasser,
(v) 0,2 bis 0,5 Gew.-% Dexpanthenol und
(vi) 0,01 bis 0,06 Gew.-% Aroma enthält, jeweils bezogen auf die Gesamtmasse der Zusammensetzung.

21. Verpackungseinheit, enthaltend ein Gefäß mit einer Zusammensetzung gemäß.einem der Ansprüche 1 bis 20, eine Schale zur Aufnahme der zu einer einmaligen Behandlung der Zähne erforderlichen Menge und einen Applikator für die Zusammensetzung.

22. Verwendung einer Zusammensetzung gemäß den Ansprüchen 1 bis 20 zum Bleichen von Zähnen.

23. Verwendung einer Zusammensetzung gemäß den Ansprüchen 1 bis 20 zur Herstellung eines Mittels zum Bleichen von Zähnen.

24. Verwendung nach Anspruch 22 oder 23 zum Bleichen von Zähnen durch zweimal tägliche Applikation.

25. Verwendung nach Anspruch 24, wobei die Einwirkzeit pro Applikation jeweils 15 bis 30 Minuten beträgt.

26. Verwendung nach Anspruch 24 oder 25 wobei sich die Gesamtanwendungsdauer über einen Zeitraum von vierzehn Tagen erstreckt.

## Claims

1. An oral composition comprising:
(i) 70 - 90 wt.% solvent,
(ii) 2 - 20 wt.% of a pharmaceutically acceptable oxidising agent and
(iii) 8 - 12 wt.% alkyl cellulose, each based on the total mass of the composition.

2. The composition according to claim 1, additionally comprising (iv) pharmaceutically acceptable acid.

3. The composition according to claim 2, additionally comprising citric acid.

4. The composition according to claim 2 or 3, comprising 0.05 - 5.0 wt.% acid, based on the total mass of the composition.

5. The composition according to one of claims 1 to 4, additionally comprising dexpanthenol.

6. The composition according to claim 5, comprising 0.1 to 1.0 wt.% dexpanthenol, based on the total mass of the composition.

7. The composition according to one of claims 1 to 6, additionally comprising (vi) flavouring substance, flavour modifier and/or edulcorant.

8. The composition according to one of claims 1 to 7, comprising ethanol, diethyl ether, ethyl acetate and/or acetone as the solvent.

9. The composition according to claim 8, comprising ethanol as the solvent.

10. The composition according to one of claims 1 to 9, comprising an alkyl cellulose having a water-solubility at room temperature of maximum 0.1 g in 100 g water as the alkyl cellulose.

11. The composition according to one of claims 1 to 10, comprising an alkyl cellulose having a viscosity of 50 to 150 mPa s, measured as a 5 wt.% solution in toluene/ethanol at 25 °C with an Ubbelohde viscosimeter.

12. The composition according to claim 10 or 11, comprising ethyl cellulose as the alkyl cellulose.

13. The composition according to one of claims 1 to 12, comprising mint oil as the flavouring substance.

14. The composition according to one of claims 1 to 13, comprising a peroxide compound as the pharmaceutically acceptable oxidising agent.

15. The composition according to one of claims 1 to 14, comprising
(i) 70.0 to 90.0 wt.% ethanol,
(ii) 2 to 16.0 wt.% oxidising agent,
(iii) 8.0 to 12.0 wt.% ethyl cellulose,
(iv) 0 to 5.0 wt.% citric acid,
(v) 0 to 1.0 wt.% dexpanthenol,
(vi) 0 to 0.5 wt.% aroma, each based on the total mass of the composition.

16. The composition according to claim 14 or 15, comprising carbamide peroxide as the peroxide compound.

17. The composition according to claim 16, comprising
(i) 79 to 85 wt.% ethanol,
(ii) 5.3 to 6.5 wt.% carbamide peroxide,
(iii) 8.5 to 9.5 wt.% ethyl cellulose,
(iv) 0 or 0.3 to 0.6 wt.% citric acid,
(v) 0.2 to 0.5 wt.% dexpanthenol and
(vi) 0.01 to 0.06 wt.% aroma, each based on the total mass of the composition.

18. The composition according to claim 14 or 15, comprising hydrogen peroxide as the peroxide compound.

19. The composition according to claim 18, comprising up to 30 wt.% water, based on the total amount of solvent.

20. The composition according to claim 18 or 19, comprising
(i) 55 to 72 wt.% ethanol,
(ii) 5.5 to 13.0 wt.% hydrogen peroxide,
(iii) 8.5 to 9.5 wt.% ethyl cellulose,
(iv) 15 to 28 wt.% water,
(v) 0.2 to 0.5 wt.% dexpanthenol and
(vi) 0.01 to 0.06 wt.% aroma, each based on the total mass of the composition.

21. A packaging unit comprising a container with a composition according to one of claims 1 to 20, a bowl for receiving the amount required for a single treatment of the teeth and an applicator for the composition.

22. Use of a composition according to one of claims 1 to 20 for bleaching teeth.

23. Use of a composition according to one of claims 1 to 20 for producing an agent for bleaching teeth.

24. Use according to claim 22 or 23 for bleaching teeth by a twice daily application.

25. Use according to claim 24, wherein the contact time for each application is 15 to 30 minutes.

26. Use according to claim 24 or 25, wherein the total duration of the application extends over a period of fourteen days.

## Revendications

1. Composition orale contenant :
i) 70 à 90 % en poids d'un solvant,
ii) 2 à 20 % en poids d'un oxydant pharmacologiquement admissible,
iii) et 8 à 12 % en poids d'une alkyl-cellulose,
par rapport, dans chaque cas, au poids total de la composition.

2. Composition conforme à la revendication 1, qui contient en plus (iv) un acide pharmacologiquement admissible.

3. Composition conforme à la revendication 2, qui contient en plus de l'acide citrique.

4. Composition conforme à la revendication 2 ou 3, qui contient 0,05 à 5,0 % en poids d'acide, par rapport au poids total de la composition.

5. Composition conforme à l'une des revendications 1 à 4, qui contient en plus (v) du dexpanthénol.

6. Composition conforme à la revendication 5, qui contient 0,1 à 1,0 % en poids de dexpanthénol, par rapport au poids total de la composition.

7. Composition conforme à l'une des revendications 1 à 6, qui contient en outre (vi) un aromatisant, un agent de sapidité et/ou un édulcorant.

8. Composition conforme à l'une des revendications 1 à 7, qui contient, en tant que solvant, de l'éthanol, de l'éther diéthylique, de l'acétate d'éthyle et/ou de l'acétone.

9. Composition conforme à la revendication 8, qui contient, en tant que solvant, de l'éthanol.

10. Composition conforme à l'une des revendications 1 à 9, qui contient, en tant qu'alkyl-cellulose, une alkyl-cellulose dont la solubilité maximale dans l'eau à température ambiante vaut de 0,1 à 100 g.

11. Composition conforme à l'une des revendications 1 à 10, qui contient une alkyl-cellulose dont la viscosité, mesurée à 25 °C au moyen d'un viscosimètre d'Ubbelohde sur une solution à 5 % en poids dans un mélange toluène/éthanol 80/20 en volume, vaut de 50 à 150 mPa.s.

12. Composition conforme à la revendication 10 ou 11, qui contient, en tant qu'alkyl-cellulose, une éthyl-cellulose.

13. Composition conforme à l'une des revendications 7 à 12, qui contient, en tant qu'aromatisant, de l'huile essentielle de menthe.

14. Composition conforme à l'une des revendications 1 à 13, qui contient, en tant qu'oxydant pharmacologiquement admissible, un composé de type peroxyde.

15. Composition conforme à l'une des revendications 1 à 14, qui contient :
i) 70,0 à 90,0 % en poids d'éthanol,
ii) 2 à 16,0 % en poids d'un oxydant,
iii) 8,0 à 12,0 % en poids d'éthyl-cellulose,
iv) 0 à 5,0 % en poids d'acide citrique,
v) 0 à 1,0 % en poids de dexpanthénol,
vi) et 0 à 0,5 % en poids d'aromatisant,
par rapport, dans chaque cas, au poids total de la composition.

16. Composition conforme à la revendication 14 ou 15, qui contient, en tant que peroxyde, du peroxyde de carbamide.

17. Composition conforme à la revendication 16, qui contient :
i) 79 à 85 % en poids d'éthanol,
ii) 5,3 à 6,5 % en poids de peroxyde de carbamide,
iii) 8,5 à 9,5 % en poids d'éthyl-cellulose,
iv) 0 ou 0,3 à 0,6 % en poids d'acide citrique,
v) 0,2 à 0,5 % en poids de dexpanthénol,
vi) et 0,01 à 0,06 % en poids d'aromatisant,
par rapport, dans chaque cas, au poids total de la composition.

18. Composition conforme à la revendication 14 ou 15, qui contient, en tant que peroxyde, du peroxyde d'hydrogène.

19. Composition conforme à la revendication 18, qui contient jusqu'à 30 % en poids d'eau, par rapport à la quantité totale de solvant.

20. Composition conforme à la revendication 18 ou 19, qui contient :
i) 55 à 72 % en poids d'éthanol,
ii) 5,5 à 13,0 % en poids de peroxyde d'hydrogène,
iii) 8,5 à 9,5 % en poids d'éthyl-cellulose,
iv) 15 à 28 % en poids d'eau,
v) 0,2 à 0,5 % en poids de dexpanthénol,
vi) et 0,01 à 0,06 % en poids d'aromatisant,
par rapport, dans chaque cas, au poids total de la composition.

21. Paquet unitaire qui comprend un récipient contenant une composition conforme à l'une des revendications 1 à 20, une coupelle servant à en prendre la quantité nécessaire pour une opération de traitement des dents, et un instrument servant à appliquer la composition.

22. Utilisation d'une composition conforme à l'une des revendications 1 à 20 pour le blanchiment des dents.

23. Utilisation d'une composition conforme à l'une des revendications 1 à 20 pour la préparation d'un agent de blanchiment des dents.

24. Utilisation conforme à la revendication 22 ou 23 pour le blanchiment des dents, par application deux fois par jour.

25. Utilisation conforme à la revendication 24, dans laquelle, lors de chaque application, on laisse agir 15 à 30 minutes.

26. Utilisation conforme à la revendication 24 ou 25, dans laquelle la durée totale d'utilisation s'étend sur un laps de temps de quatorze jours.
